# EUROPEAN PATENT APPLICATION

(11) **EP 4 843 477 A1**
(43) Date of publication of application: **25.11.2026**
(21) Application number: 26177922.7
(22) Date of filing: 11.05.2026
(51) Int. Cl.: A61B 3/10, G01J 9/00

(54) **WAVEFRONT SENSOR**

(30) Priority: 23.05.2025 JP 2025086557
(71) Applicant: Tamron Co., Ltd., Saitama-shi Saitama 337-8556 (JP)
(72) Inventor: MIYAGAWA, Suguru, Saitama-shi, 337-8556 (JP)
(74) Representative: MFG Patentanwälte Meyer-Wildhagen Meggle-Freund Gerhard PartG mbB

(57) **Abstract**

A wavefront sensor (1300) measures wavefront aberration of target light, and includes: an afocal optical system that relays the target light, the afocal optical system including one or more negative lenses; a microlens array (1310) that acts on the target light transmitted through the afocal optical system to form a focal spot group; and an image sensor (1311) that generates an image representing the focal spot group formed by the microlens array (1310).

## Description

### CROSS-REFERENCE TO RELATED APPLICATIONS

This application is based on and claims the benefit of priority from Japanese Patent Application No. 2025-086557, filed on May 23, 2025, the entire contents of which are incorporated herein by reference.

### BACKGROUND OF THE INVENTION

### Technical Field

The present invention relates to a wavefront sensor.

### Related Art

A Shack-Hartmann type wavefront aberration measuring apparatus (wavefront sensor) using a microlens array has been used for industrial use and medical use. In particular, for medical use, it is used for ophthalmology clinic and eyeglass shops, measures optical aberrations of a human eye, and is widely used for prescriptions of eyeglasses and contact lenses. In a conventional ophthalmic wavefront sensor, or in a conventional autorefractometer that examines an optical state of a human eye using another principle, a large stationary-type apparatus has been widely prevalent.

In most cases, measurement in such a widely prevalent apparatus is performed while a subject looks into the apparatus and fixates on an artificially created fixation target through an internal optical system. An examination must be performed in a state in which the ciliary muscle of a human eye is most relaxed, that is, a state in which focus is adjusted to the farthest distance, and an image that appears to be located at a far distance is used as the fixation target to be fixated, and, optically as well, focusing to the far distance is encouraged by repeating defocus. Such a mechanism is referred to as fogging method; however, since it is a method of looking in with one eye, there is a demerit that there is a case where a sense of depth cannot be properly obtained and focus cannot be adjusted to the far distance, and since the fixation target is optically defocused, this leads to complication and upsizing of the apparatus and an increase in manufacturing cost, and also measurement time is required due to the time required for defocus.

Therefore, apparatuses called open-view type apparatuses, in one of which the subject can see a scenery on the other side via the apparatus through a mirror and an optical system, have been devised, and some of the apparatuses have actually been commercially available. Such an open-view type apparatus does not require an optical system for the fogging method, and can be expected to be downsized, avoid a phenomenon in which a sense of depth cannot be obtained, and also shorten measurement time.

With an aim of further downsizing and cost reduction by eliminating movable parts, research has been conducted to implement an open-view type ophthalmic wavefront sensor using a Shack-Hartmann type wavefront sensor with a microlens array, and the wavefront sensor has been actually commercialized (JP 2018-501936 A and JP 2016-526985 A).

However, in a Shack-Hartmann type wavefront sensor, there has been a problem that a working distance of a relay optical system disposed in front of a microlens array is extremely short. For this reason, in an ophthalmic wavefront sensor in which a Shack-Hartmann type wavefront sensor is specialized for ophthalmic use, there has arisen a problem that the wavefront sensor can be used only in a state in which the apparatus is brought extremely close to the subject's eye and is in close contact with the face.

One aspect of the present invention has been made in view of the above problem, and an object thereof is to provide a wavefront sensor capable of increasing a working distance of a relay optical system disposed in front of a microlens array while maintaining an optical total length of the relay optical system.

### SUMMARY OF THE INVENTION

In order to solve the above problem, a wavefront sensor according to one aspect of the present invention measures wavefront aberration of target light, and includes: an afocal optical system that relays the target light, the afocal optical system including one or more negative lenses; a microlens array that acts on the target light transmitted through the afocal optical system to form a focal spot group; and an image sensor that generates an image representing the focal spot group formed by the microlens array.

### Advantageous Effects of Invention

According to one aspect of the present invention, in a wavefront sensor in which a relay optical system is disposed in front of a microlens array, it is possible to increase a working distance of the relay optical system while maintaining an optical total length of the relay optical system.

### BRIEF DESCRIPTION OF THE DRAWINGS

Fig. 1 is a schematic diagram of a conventional ophthalmic wavefront sensor;
Fig. 2 is a diagram showing an example in which a myopic eye is measured;
Fig. 3 is a diagram showing an example in which a hyperopic eye is measured;
Fig. 4 is a diagram showing an optical system obtained by removing a relay optical system from the ophthalmic wavefront sensor shown in Fig. 1;
Fig. 5 is a diagram showing an afocal optical system not including a negative lens;
Fig. 6 is a side view showing an example of a composite lens including a negative lens;
Fig. 7 is a diagram showing an afocal optical system including a negative lens;
Fig. 8 is a diagram showing optical paths of a collimated beam, a converging beam, and a diverging beam incident on the afocal optical system shown in Fig. 5 (the afocal optical system not including a negative lens);
Fig. 9 is a diagram showing optical paths of a collimated beam, a converging beam, and a diverging beam incident on the afocal optical system shown in Fig. 7 (the afocal optical system including a negative lens);
Fig. 10 is a diagram showing a relationship among a working distance, a spacing between lenses, an optical total length, and a distance from a rearmost lens to a final image in the afocal optical system shown in Fig. 7 (the afocal optical system including a negative lens);
Fig. 11 is a diagram showing optical paths of a collimated beam, a converging beam, and a diverging beam incident on an afocal optical system that is the afocal optical system shown in Fig. 5 and in which a lens configuration is changed so that a distance from an object to the afocal optical system matches that of the afocal optical system shown in Fig. 7;
Fig. 12 is a diagram showing optical paths of a collimated beam, a converging beam, and a diverging beam incident on an afocal optical system that is the afocal optical system shown in Fig. 5 and in which a lens configuration is changed so that a distance from the afocal optical system to a final image object matches that of the afocal optical system shown in Fig. 7;
Fig. 13 is a diagram showing an example of a configuration of a wavefront sensor according to one embodiment of the present invention; and
Fig. 14 is a diagram showing an example of a configuration of an ophthalmic wavefront sensor according to one embodiment of the present invention.

### DESCRIPTION OF THE EMBODIMENTS

### [Problems to be Solved in the Embodiment]

In a conventional ophthalmic wavefront sensor, or in a conventional autorefractometer that examines an optical state of a human eye using another principle, a large stationary-type apparatus has been widely prevalent. A conventional large autorefractometer has a complicated optical system inside. For example, an autorefractometer called a focusing type projects ring-shaped light onto a fundus of a subject. It has a mechanism of measuring an optical state of a human eye by forming an image of light reflected from the fundus on an image sensor and analyzing the formed ring-shaped image. At this time, in order to form the ring-shaped projected image on the image sensor, a focusing function for moving a lens of the internal optical system is required. Further, many autorefractometers have a structure in which the subject looks into the inside of the apparatus through an objective lens, and present an image serving as a fixation target to the subject through an optical system. Since it is desired to prompt the subject to focus to a far distance, the image serving as the fixation target is repeatedly defocused. An optical system for defocusing this fixation target is required as a separate system from the measurement optical system.

In order to implement such a complicated optical system and operation, a conventional autorefractometer has a large size and a complicated internal structure. Further, since the subject looks into the inside of the apparatus with one eye and is shown a pseudo image, a state at the time of measurement is also not a natural state.

In order to solve these problems of conventional autorefractometers, an apparatus has been researched and developed in which movable parts are eliminated by employing, as a measurement system, a wavefront sensor using a microlens array, an optical system is downsized, and, instead of a structure in which the subject looks into the apparatus, the subject can view a fixation target existing in a distant real space during measurement.

According to the technique of JP 2018-501936 A described above, it has a structure inside which a measurement optical system incorporates a small wavefront sensor, and by which the subject can see through to the other side via the apparatus by devising the internal optical system. JP 2016-526985 A also differs in the internal optical system, but is common in having a structure enabling seeing through to the other side through the apparatus. Further, since each apparatus is small, the apparatus does not block a line of sight of an eye on a side opposite to the eye being measured, and it is possible to view a distant fixation target with both eyes.

However, the techniques of JP 2018-501936 A and JP 2016-526985 A each have an extremely short working distance, and can only be used in such a manner that the apparatus is brought into close contact with the eye. Further, since the apparatus has a structure in which the other side is seen through an internal optical system of the apparatus and a narrow aperture, it cannot be said that measurement is performed with both eyes spatially open.

Further, when prescribing eyeglasses, in order to measure whether a power of the eyeglasses is correct, measurement may be performed through a lens of the eyeglasses. However, in a mode in which the apparatus is used while being brought into close contact with an eye, there is no space for an eyeglass lens to be interposed, and therefore such measurement cannot be performed.

A structure in which the apparatus is brought into close contact also has a hygienic problem because it touches an unspecified large number of subjects, and further, when the subject is not accustomed to handling the apparatus, there is also a risk that an accident may be caused by the apparatus coming into contact with the face or the eyeball.

In order to solve such technical problems, the inventor of the present application has devised a structure that significantly extends a working distance of the apparatus while downsizing an optical system.

### [Optical Principle of Ophthalmic Wavefront Sensor]

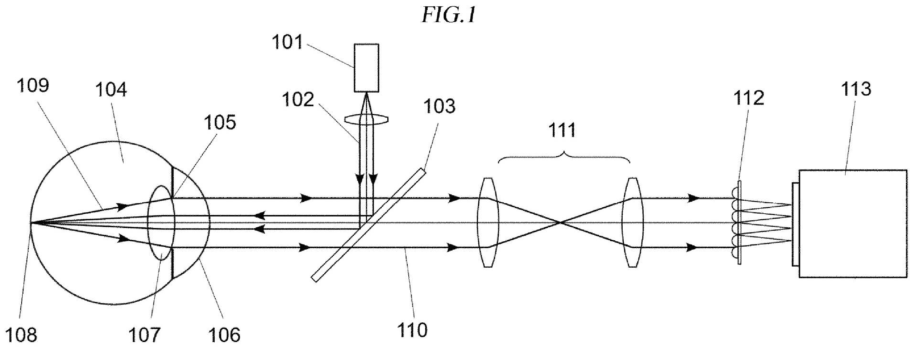

Before describing a configuration of the wavefront sensor according to the present embodiment, an optical principle of a conventional ophthalmic wavefront sensor will be described. Fig. 1 is a schematic diagram of the conventional ophthalmic wavefront sensor. First, light 102 emitted from a light source 101 is reflected by a half mirror 103 and travels toward an eye 104 which is a measurement target. The light 102 is monochromatic light such as laser light, and is a beam having a small diameter. The light 102 enters the eye 104 from a pupil 105, is refracted by a cornea 106 and a crystalline lens 107, and reaches a fundus 108. The light 102 having reached the fundus 108 is diffusely reflected at the fundus 108, thereby behaving as if it were a point light source.

Light emitted from a point light source can be assumed to be a spherical wave. Light 109 that has become a spherical wave passes through the crystalline lens 107 and the cornea 106, and exits to the outside from the pupil 105. Light 110 that has exited to the outside becomes any of a diverging light, a converging light, and a collimated light, in accordance with an optical refractive power of the subject's eye.

The light 110, which is reflected light from the eye, is transmitted through a relay optical system 111 and is irradiated onto a microlens array 112. The microlens array 112 focuses the light 110 onto an image sensor 113 to form a point image. A principle of measuring wavefront aberration based on an amount of movement of this point image is the same as that of a general Shack-Hartmann type wavefront sensor.

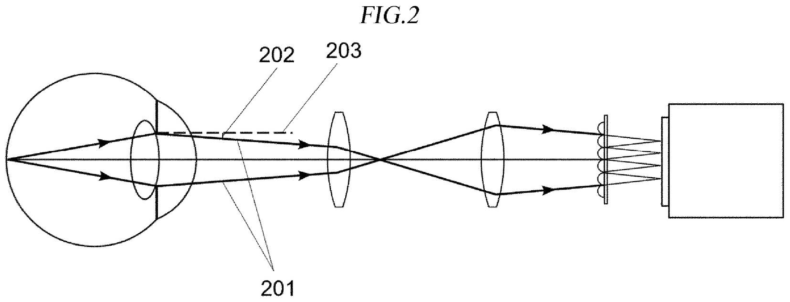

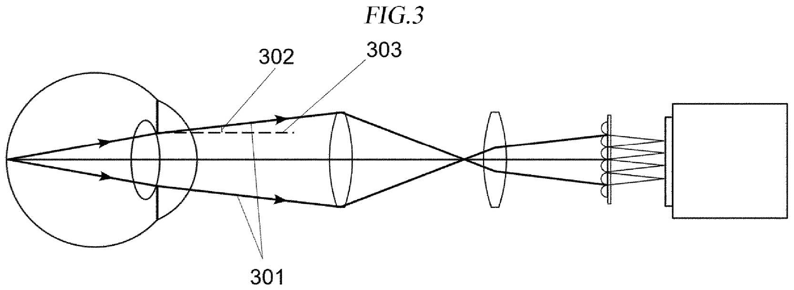

Fig. 2 shows an example in which a myopic eye is measured. However, a light source unit in the drawing is omitted. When light for measurement is made incident on the eye from the pupil in the same manner as described above, in a case that the subject is myopic, light emitted from the eye becomes a converging light as indicated by light 201 in Fig. 2. An angle at which the light converges differs depending on a degree of myopia, and as the degree of myopia becomes stronger, a convergence angle 202 of the light becomes larger. (Here, a dotted line 203 is a straight line parallel to an optical axis.) Fig. 3 shows an example in which a hyperopic eye is measured. Contrary to the case of myopia, light 301 emitted from the eye becomes a diverging light. As the degree of hyperopia becomes stronger, a divergence angle 302 of the light becomes larger. (Here, the dotted line 303 is a straight line parallel to an optical axis.)
Note that Fig. 1 shows an example in which an emmetropic eye is measured. Emmetropia refers to a state in which an object at infinity is imaged on a fundus, and the light 110 emitted from the eye becomes a collimated light.

There are also types such as astigmatism and irregular astigmatism; however, since an influence on a trajectory of light rays is smaller than that of myopia or hyperopia, for describing the present embodiment, it may be considered that a refractive state of a human eye is roughly classified into these three types.

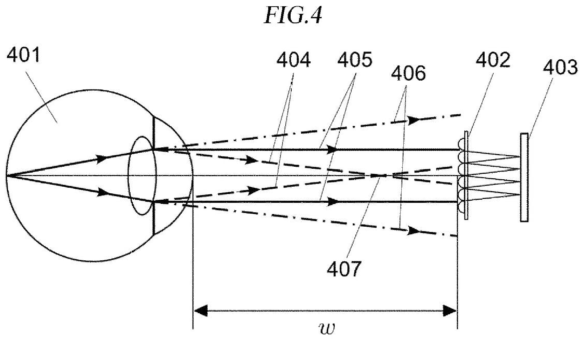

Here, considering downsizing the ophthalmic wavefront sensor of Fig. 1, an optical system (Fig. 4) in which the relay optical system 111 is removed is considered. When the light source 101 for measurement and the half mirror 103 therebetween are omitted, as shown in Fig. 4, an optical system constituted only of a microlens array 402 and an image sensor 403 can be considered. A distance from an eye 401 to the microlens array 402 is a finite distance and is a spatial distance w that is not 0.

At this time, light emitted from the eye becomes a converging light 404 in a case of myopia, a collimated light 405 in a case of emmetropia, and a diverging light 406 in a case of hyperopia. Areas on which these lights fall on the microlens array 402 are different. In particular, in a case of a converging light, depending on a position of the microlens array 402, there is a position (a position of a point 407) where the area becomes extremely small, and in such a case, measurement becomes impossible. Further, when the microlens array is located behind the point 407 at which an upper ray and a lower ray of the converging light intersect, the light becomes a diverging light, and it is not possible to correctly measure the converging light that is actually to be measured. Further, also in a case of hyperopia in which the light becomes a diverging light, when a distance between the eye and the microlens array is long, the light diverges, and thus light incident on the microlens array is only a very small part of the light emitted from the eye, and a point image formed on the image sensor becomes extremely dark, and thus, measurement may also become impossible.

In order to avoid such a situation, it is necessary either to bring the microlens array extremely close to the eye in order to reduce the distance w, or to provide a relay optical system therebetween. When trying to measure strong myopia, it is assumed that the point 407 is at a position extremely close to the eye, and the distance w becomes a very small value. A method of bringing the microlens array extremely close to the eye results in bringing the apparatus into close contact with the eye, which is contrary to the problem to be solved by the present embodiment.

Therefore, by devising a design of the relay optical system therebetween, it is considered to determine conditions under which measurement becomes possible regardless of whether the eye to be measured is myopic or hyperopic, and also regardless of the degree thereof, and under which it is possible to secure a sufficient distance between the relay optical system and the objective lens.

When considering a wavefront sensor, an optical system that relays light must not affect curvature of a wavefront of the light before and after relaying. For example, when considering evaluating collimated light, if light incident on the optical system is collimated light, light emitted from the optical system must also be collimated light. Such an optical system is referred to as afocal optical system, and is widely used in optical systems such as a telescope and a beam expander, and is a known technique.

Here, considering again the optical system of Fig. 4, it has already been described that, when there is no relay optical system, the closer the distance w between the microlens array and the eye (pupil) is, the less the influence of convergence/divergence of light is. If it is considered to implement this by inserting a relay optical system therebetween, it is sufficient that an image of the pupil is formed so as to be located on the microlens array. That is, by forming the image of the pupil on the microlens array, it can be regarded that, when viewed from the microlens array, this is equivalent to a case where the distance w is 0.

When considering an arbitrary afocal optical system, the optical system does not have a conjugate point with respect to an object at infinity, but it is known that, with respect to an object at a finite distance, if conditions are satisfied, the optical system has a conjugate point at a finite distance behind the optical system. Such a lens system is called a bi-telecentric lens, and is a known technique used for precision measurement of dimensions and the like.

### [Afocal Optical System]

A lens optical system that satisfies an afocal condition is referred to as "afocal optical system". In the present description, the "afocal condition" refers to a condition that, when a light beam incident on a lens system from the front side is a collimated beam, a light beam emitted rearward from the lens system is also a collimated beam, and, when a light beam incident on the lens system from the rear side is a collimated beam, a light beam emitted forward from the lens system is also a collimated beam.

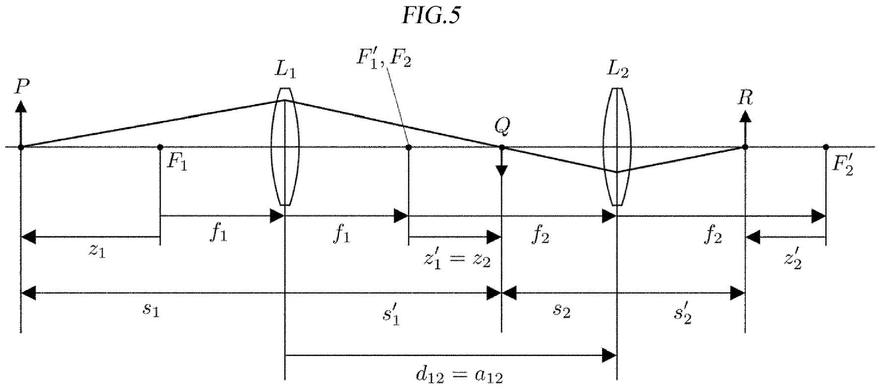

An afocal optical system can be constituted of two lens groups having positive power. In the present description, a lens group on an object side is referred to as "front group", and a lens group on an image side is referred to as "rear group". The optical system shown in Fig. 5 is an afocal optical system in which the front group is constituted of one positive lens L1 (hereinafter, also simply referred to as lens L1), and the rear group is constituted of one positive lens L2 (hereinafter, also simply referred to as lens L2). Here, the "positive lens" refers to a convex lens and other lenses having positive power.

Hereinafter, a focal length of the lens L1 is defined as f1 (>0), and a focal length of the lens L2 is defined as f2 (>0). Further, a spacing between an image-side principal point of the lens L1 and an object-side principal point of the lens L2 (hereinafter, also referred to as "principal point spacing") is defined as d12. Note that it is assumed that the lens L1 and the lens L2 are thin lenses each having a negligible thickness. In this case, an object-side principal point and an image-side principal point of the lens L1 coincide with a center of the lens L1, and an object-side principal point and an image-side principal point of the lens L2 coincide with a center of the lens L2. Further, an optical total length a12, that is, a distance from a vertex of an incident surface of the lens L1 to a vertex of an exit surface of the lens L2, coincides with the principal point spacing d12 between the lens L1 and the lens L2. Note that, in Fig. 5, a positive quantity having a dimension of distance is represented by a rightward arrow, and a negative quantity having a dimension of distance is represented by a leftward arrow.

Hereinafter, all conditional expressions of the lens system are based on paraxial approximation calculation, and only powers of positive and negative lenses are taken into consideration, and aberrations of the lenses are not taken into consideration.

In the lens system shown in Fig. 5, the afocal condition is expressed by the following Conditional Expression (1).
[Expression 1] ${d}_{12} = {f}_{1} + {f}_{2}$ (1)

Assuming that the lens L1 forms, behind the lens L1, an image Q of an object P (hereinafter, referred to as intermediate image Q) disposed in front of the lens L1, a distance from the lens L1 to the object P is defined as s1 (<0), and a distance from an object-side focal point F1 of the lens L1 to the object P is defined as z1 (<0). Further, a distance from the lens L1 to the intermediate image Q is defined as s1' (>0), and a distance from an image-side focal point F1' of the lens L1 to the intermediate image Q is defined as z1' (>0). Then, for the lens L1, the following Relational Expression (2) is obtained from Newton's imaging equation.
[Expression 2] ${z}_{1} {z}_{1}^{′} = - {f}_{1}^{2}$ (2)

Further, assuming that the lens L2 forms, behind the lens L2, an image R of the intermediate image Q (hereinafter, referred to as final image R) disposed in front of the lens L2, a distance from the lens L2 to the intermediate image Q is defined as s2 (<0), and a distance from an object-side focal point F2 of the lens L2 to the intermediate image Q is defined as z2 (>0). Further, a distance from the lens L2 to the final image R is defined as s2' (>0), and a distance from an image-side focal point F2' of the lens L2 to the final image R is defined as z2' (<0). Then, for the lens L2, the following Relational Expression (3) is obtained from Newton's imaging equation.
[Expression 3] ${z}_{2} {z}_{2}^{′} = - {f}_{2}^{2}$ (3)

When the afocal condition is satisfied, a position of the image-side focal point F1' of the lens L1 coincides with a position of the object-side focal point F2 of the lens L2. Therefore, the following Expression (4) is derived.
[Expression 4] ${z}_{2} = {z}_{1}^{′}$ (4)

Based on Expression (2), Expression (3), and Expression (4), the following Expression (5) is obtained.
[Expression 5] ${z}_{2}^{′} = - \frac{{f}_{2}^{2}}{{z}_{2}} = - \frac{{f}_{2}^{2}}{{z}_{1}^{′}} = {\left(\frac{{f}_{2}}{{f}_{1}}\right)}^{2} {z}_{1}$ (5)

In the present description, "rear imaging condition" refers to a condition imposed on a position of the object P so that the final image R is formed behind the afocal optical system. Hereinafter, the rear imaging condition in the afocal optical system shown in Fig. 5 will be examined.

When the final image R is formed behind the afocal optical system, a distance s2' from the lens L2 to the final image R satisfies the following Conditional Expression (6).
[Expression 6] ${s}_{2}^{′} > 0$ (6)

Noting that, in addition to Expression (5), the following Expression (7) holds from Fig. 5, Conditional Expression (6) can be transformed into the following Conditional Expression (8).
[Expression 7] ${s}_{2}^{′} = {f}_{2} + {z}_{2}^{′}$ (7) [Expression 8] $\frac{{f}_{1}^{2} {f}_{2} + {f}_{2}^{2} {z}_{1}}{{f}_{1}^{2}} > 0$ (8)

Noting that f1 > 0, Conditional Expression (8) can be transformed into the following Conditional Expression (9).
[Expression 9] ${f}_{1}^{2} {f}_{2} + {f}_{2}^{2} {z}_{1} > 0$ (9)

Further, noting from Fig. 5 that s1 = -f1 + z1, Conditional Expression (9) can be transformed into the following Conditional Expression (10).
[Expression 10] ${f}_{1}^{2} + {f}_{2} \left({s}_{1}+{f}_{1}\right) > 0$ (10)

When Conditional Expression (1) is substituted into Conditional Expression (10) and rearranged, the following Conditional Expression (11) is obtained as a conditional expression that the distance s1 from the lens L1 to the object P should satisfy.
[Expression 11] $\frac{{f}_{1}}{{f}_{2}} {d}_{12} > - {s}_{1}$ (11)

A distance -s1 from the object P to the lens L1 is referred to as "working distance". As can be seen from Conditional Expression (11), in order to satisfy the rear imaging condition, the working distance -s1 cannot be made equal to or greater than (f1/f2) × d12. As an example, in a case where f1 = f2 is satisfied, the working distance -s1 cannot be made equal to or greater than the principal point spacing d12. Therefore, when the optical total length a12 cannot be made sufficiently long due to reasons such as space constraints, that is, when the principal point spacing d12 between the lenses L1 and L2 cannot be made sufficiently large, the working distance -s1 also cannot be made sufficiently large.

In the techniques described in JP 2018-501936 A and JP 2016-526985 A, in each case, the design is such that the eye is used while being brought extremely close to an objective lens. It is considered that this is due to such optical constraints.

### [Afocal Optical System Including Negative Lens]

As described above, in the afocal optical system shown in Fig. 5, when the optical total length a12 cannot be made sufficiently long, the working distance -s1 cannot be made sufficiently large. In other words, in order to make the working distance -s1 sufficiently large, it is necessary to make the optical total length a12 sufficiently long. Such a constraint condition can be mitigated by including a negative lens in the afocal optical system. Here, "negative lens" refers to a concave lens and other lenses having negative power.

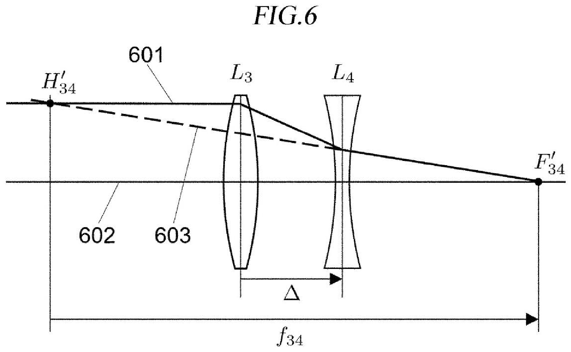

First, a composite lens including a negative lens will be described with reference to Fig. 6. Fig. 6 is a side view showing an example of a composite lens including a negative lens.

The composite lens shown in Fig. 6 is constituted of a positive lens L3 (hereinafter, also simply referred to as lens L3) and a negative lens L4 (hereinafter, also simply referred to as lens L4) sharing an optical axis 602. Here, the composite lens refers to a virtual lens in which two or more lenses are regarded as one lens.

Consider that a ray 601 parallel to the optical axis 602 is incident on the lens L3. The ray 601 is refracted by the lens L3 and travels toward the lens L4. Then, the ray 601 refracted by the lens L3 is further refracted by the lens L4, and intersects the optical axis 602. An intersection point between an optical path of the ray 601 refracted by the lens L4 and the optical axis 602 is an image-side focal point F34' of the composite lens.

Further, by linearly extending the optical path of the ray 601 that is refracted by the lens L4 and travels toward the image-side focal point F34', an extension line 603 is defined. An intersection point between the optical path of the ray 601 before being incident on the lens L3 and the extension line 603 is an image-side principal point H34' of the composite lens. A composite focal length f34 of the composite lens is a distance from the image-side principal point H34' to the image-side focal point F34' measured along the optical axis 602. The composite focal length f34 is obtained by the following Expression (12), wherein a focal length of the lens L3 is defined as f3, a focal length of the lens L4 is defined as f4, and a spacing between the lens L3 and the lens L4 is defined as Δ.
[Expression 12] ${f}_{34} = \frac{{f}_{3} {f}_{4}}{{f}_{3} + {f}_{4} - Δ}$ (12)

The composite lens shown in Fig. 6 is optically equivalent to a positive lens having a focal length of f34 and disposed at the position H34'. In the composite lens shown in Fig. 6, the image-side principal point H34' is located on an object side of the lens L3, and thus, the optical total length can be shortened as compared with the composite focal length f34. Such a composite lens is widely used in, for example, a telephoto lens.

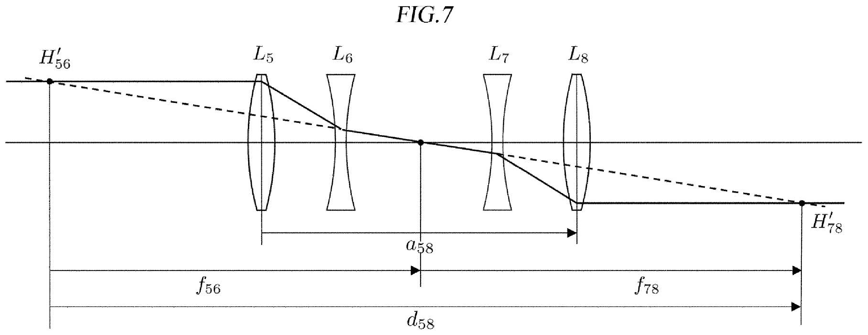

Next, an afocal optical system including a negative lens will be described with reference to Fig. 7. Fig. 7 is a side view showing an example of the afocal optical system including the negative lens.

The lens system shown in Fig. 7 is an afocal optical system in which a front group is constituted of the composite lens shown in Fig. 6, and a rear group is constituted of a composite lens obtained by reversing the composite lens shown in Fig. 6 in a front-rear direction. In Fig. 7, a positive lens and a negative lens constituting the front group are respectively a lens L5 and a lens L6. Further, a negative lens and a positive lens constituting the rear group are respectively a lens L7 and a lens L8. Hereinafter, a composite focal length of the front group is defined as f56, a focal length of the rear group is defined as f78, an image-side principal point of the front group is defined as H56', and an object-side principal point of the rear group is defined as H78'. The lenses L5, L6, L7, and L8 are respectively examples of a first lens, a second lens, a third lens, and a fourth lens according to the present disclosure.

As described above, the front group is equivalent to a positive lens disposed at the image-side principal point H56', and the rear group is equivalent to a positive lens disposed at the object-side principal point H78'. Therefore, when a spacing between the image-side principal point H56' of the front group and the object-side principal point H78' of the rear group (hereinafter, also referred to as "principal point spacing") is defined as d58, the afocal condition is given by the following Conditional Expression (13).
[Expression 13] ${d}_{58} = {f}_{56} + {f}_{78}$ (13)

The image-side principal point H56' of the front group is located on an object side of the lens L5 of the front group, and the object-side principal point H78' of the rear group is located on an image side of the lens L8 of the rear group. Therefore, the principal point spacing d58 between the front group and the rear group becomes larger than an optical total length a58.

Also in the afocal optical system shown in Fig. 7, in order to satisfy the rear imaging condition, a distance from the object P to the lens L5, that is, a working distance -s5, cannot be made equal to or greater than (f56/f78) × d58. However, in the afocal optical system shown in Fig. 7, even when the working distance -s5 is equal to or greater than (f56/f78) × a58, the rear imaging condition can be satisfied. As an example, in a case where f56 = f78 is satisfied, in order to satisfy the rear imaging condition, the working distance -s5 cannot be made equal to or greater than the principal point spacing d58 between the front group and the rear group. However, even when the working distance -s5 is equal to or greater than the optical total length a58, the rear imaging condition can be satisfied.

### [Operation of Afocal Optical System]

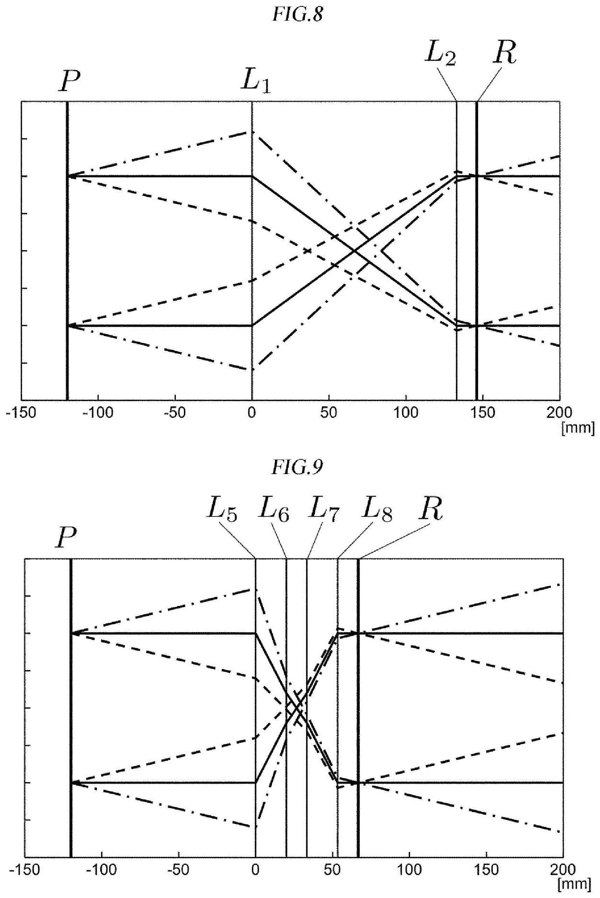

Operations of the afocal optical systems shown in Fig. 5 and Fig. 7 will be described with reference to Figs. 8 to 12.

Fig. 8 is a diagram showing optical paths of a collimated beam (solid line), a converging beam (chain line), and a diverging beam (one-dot chain line) incident on the afocal optical system shown in Fig. 5 (afocal optical system not including a negative lens). In Fig. 8, P indicates an object (position), R indicates a final image (position), L1 indicates an object-side positive lens (position), and L2 indicates an image-side positive lens (position). A horizontal axis indicates positions in which the position L1 of the object-side positive lens is defined as an origin, the object side is defined as negative, and the image side is defined as positive.

Here, for simplicity, it is assumed that the focal length f1 of the lens L1 and the focal length f2 of the lens L2 coincide with each other. Then, the principal point spacing d12 between the lenses L1 and L2 coincides with twice the focal length f1 = f2 of the lenses L1 and L2 (afocal condition). Further, for simplicity, it is assumed that the lenses L1 and L2 are thin lenses each having a negligible thickness. Then, the optical total length a12 coincides with the principal point spacing d12 between the lenses L1 and L2, and therefore coincides with twice the focal length f1 or f2 of the lenses L1 and L2. In this case, the rear imaging condition that the working distance -s1 should satisfy is given by the following Expression (14).
[Expression 14] ${d}_{12} > - {s}_{1}$ (14)

When the working distance -s1 is set to 120 mm, in order to satisfy the condition of Expression (14), it is necessary that f1 = f2 > 60 mm is satisfied. Here, it is assumed that f1 = f2 = 66.5 mm is satisfied. In this case, the final image R is formed at a position 13 mm behind the lens L2. Further, since f1 = f2 = 66.5 mm is satisfied, the optical total length a12 is 133 mm.

As is apparent from Fig. 8, in the afocal optical system shown in Fig. 5, regions in which the final image R is formed when a collimated beam (solid line), a converging beam (chain line), and a diverging beam (one-dot chain line) are incident coincide with each other. This is an advantage of satisfying the rear imaging condition. When the rear imaging condition is not satisfied, a region in which the final image R is formed changes depending on a degree of convergence/divergence of a light beam incident on the afocal optical system, and thus there is a risk of causing a hindrance to formation of a focal spot group by the microlens array or generation of image data by the image sensor.

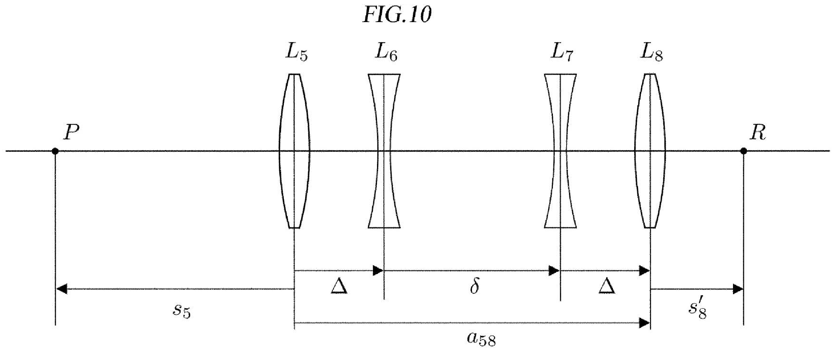

Fig. 9 is a diagram showing optical paths of a collimated beam (solid line), a converging beam (chain line), and a diverging beam (one-dot chain line) incident on the afocal optical system shown in Fig. 7 (afocal optical system including a negative lens). In Fig. 9, P indicates an object (position), R indicates a final image (position), L5 indicates a positive lens (position) of a front group, L6 indicates a negative lens (position) of the front group, L7 indicates a negative lens (position) of a rear group, and L8 indicates a positive lens (position) of the rear group. A horizontal axis indicates positions in which the position L5 of the positive lens is defined as an origin, the object side is defined as negative, and the image side is defined as positive. Here, in order to simplify calculation, it is assumed that a focal length f5 of the lens L5 and a focal length f8 of the lens L8 coincide with each other. Further, it is assumed that a focal length f6 of the lens L6 and a focal length f7 of the lens L7 coincide with each other. Further, as shown in Fig. 10, a principal point spacing between the lens L5 and the lens L6 is defined as Δ, and this is assumed to coincide with a principal point spacing between the lens L7 and the lens L8. Further, a spacing between the lens L6 and the lens L7 is defined as δ, a working distance is defined as -s5, and a distance from the lens L8 to the final image R is defined as s8'. Under such conditions, relationships among s5, s8', and the optical total length a58 are expressed by the following Expressions (15), (16), and (17).
[Expression 15] ${s}_{8} = \frac{2 {f}_{5} \left({f}_{6}-Δ\right)}{{f}_{5} + {f}_{6} - Δ} + {s}_{5}^{′}$ (15) [Expression 16] $δ = \frac{2 {f}_{6} \left({f}_{5}-Δ\right)}{{f}_{5} + {f}_{6} - Δ}$ (16) [Expression 17] ${a}_{58} = 2 Δ + δ$ (17)

In order to align conditions, arrangement of each lens and focal length of each lens are calculated by Expressions (15) and (16) such that, when the working distance -s5 is 120 mm, the distance s8' from the lens L8 to the final image R is approximately 13 mm. Then, the focal lengths f5 and f8 of the lenses L5 and L8 are 25 mm, and the focal lengths f6 and f7 of the lenses L6 and L7 are -20 mm. Further, the spacing Δ between the lens L5 and the lens L6 and the spacing Δ between the lens L7 and the lens L8 are 20 mm, and the spacing δ between the lens L6 and the lens L7 is 13.3 mm.

At this time, the composite focal length f56 of the front group and the composite focal length f78 of the rear group coincide with each other, and are each 33.3 mm based on Expression (14) for obtaining a composite focal length. Therefore, the principal point spacing d58 between the front group and the rear group is 33.3 mm × 2 = 66.6 mm. On the other hand, the optical total length a58 is 20 mm + 13.3 mm + 20 mm = 53.3 mm based on Expression (17).

As is apparent from Fig. 9, in the afocal optical system shown in Fig. 7, regions in which the final image R is formed when a collimated beam (solid line), a converging beam (chain line), and a diverging beam (one-dot chain line) are incident also coincide with each other. This is an advantage of satisfying the rear imaging condition. When the rear imaging condition is not satisfied, a region in which the image R is formed changes depending on a degree of divergence/convergence of light incident on the afocal optical system, and thus there is a risk of causing a hindrance to formation of a focal spot group by the microlens array and generation of image data by the image sensor.

When the working distance -s1 is 120 mm, the optical total length a12 of the afocal optical system shown in Fig. 5 (afocal optical system not including a negative lens) is 133 mm, whereas the optical total length a58 of the afocal optical system shown in Fig. 7 (afocal optical system including a negative lens) is 53.3 mm. That is, by including a negative lens in the afocal optical system, the optical total length can be significantly shortened while maintaining the working distance.

Next, it is considered whether or not an afocal optical system having the same working distance and optical total length as those of the afocal optical system shown in Fig. 7 can be implemented by two convex lenses.

In the afocal optical system shown in Fig. 5, an optical system (hereinafter, referred to as "comparative optical system"), in which d12 = 53.3 mm, f1 = 26.65 mm, and f2 = 26.65 mm are set, is considered. The optical total length a12 of the comparative optical system is 53.3 mm and satisfies the afocal condition. When the lenses L1 and L2 are thin lenses each having a negligible thickness, the optical total length a12 coincides with the principal point spacing d12 between the lenses L1 and L2; therefore, in order for the comparative optical system to satisfy the rear imaging condition, the working distance -s1 needs to be less than 53.3 mm (see Conditional Expression (11)).

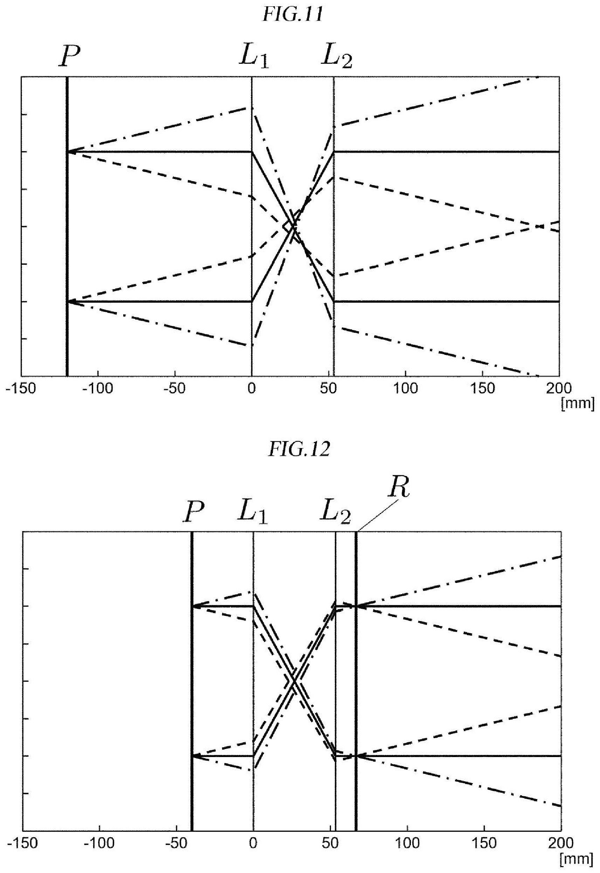

As in the afocal optical system shown in Fig. 7, Fig. 11 shows optical paths of a collimated beam (solid line), a converging beam (chain line), and a diverging beam (one-dot chain line) incident on the comparative optical system when the object P is disposed such that -s1 = 120 mm is satisfied. In this case, since the working distance -s1 is larger than the principal point spacing d12, the rear imaging condition is not satisfied. Therefore, a region in which the final image R is formed changes depending on a degree of divergence/convergence of the incident light beam, and it is not possible to exhibit a function expected of the afocal optical system. This means that, when trying to use an afocal optical system not including a negative lens at the same working distance as that of an afocal optical system including a negative lens, the rear imaging condition is not satisfied.

As in the afocal optical system shown in Fig. 7, Fig. 12 shows optical paths of a collimated beam (solid line), a converging beam (chain line), and a diverging beam (one-dot chain line) incident on the comparative optical system when the object P is disposed such that s2' = 13 mm is satisfied. In this case, since the working distance -s1 is 40 mm, the rear imaging condition is satisfied. Therefore, a region in which the final image R is formed does not change depending on a degree of divergence/convergence of the incident light beam, and it is possible to exhibit a function expected of the afocal optical system. However, the working distance -s1 of the comparative optical system is 1/3 of the working distance -s5 = 120 mm of the afocal optical system shown in Fig. 7. This means that, when an afocal optical system not including a negative lens is used while satisfying the rear imaging condition, the working distance is markedly shorter as compared with an afocal optical system including a negative lens.

### [Embodiment 1: Configuration of Wavefront Sensor]

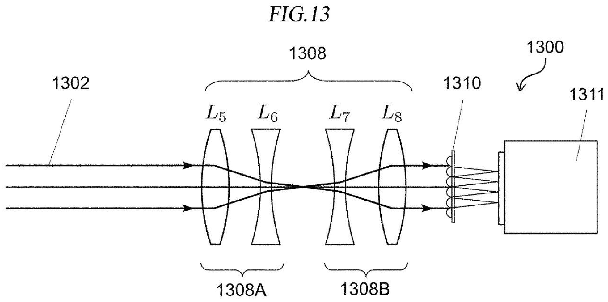

A configuration of a wavefront sensor 1300 according to the present embodiment will be described with reference to Fig. 13. Fig. 13 is a side view showing the configuration of the wavefront sensor 1300 according to the present embodiment.

The wavefront sensor 1300 is a Shack-Hartmann type wavefront sensor that measures wavefront aberration of target light 1302, and includes a relay optical system 1308, a microlens array 1310, and an image sensor 1311.

The relay optical system 1308 is a configuration for relaying the target light 1302. The microlens array 1310 is a configuration for acting on the target light 1302 transmitted through the relay optical system 1308 to form a focal spot group on the image sensor 1311. The image sensor 1311 is a configuration for generating image data representing the focal spot group formed by the microlens array 1310.

A distribution of the focal spot group formed by the microlens array 1310 is determined in accordance with wavefront aberration. Therefore, by analyzing the image data generated by the image sensor 1311 using a computer which is not shown, it is possible to calculate various physical quantities representing wavefront aberration of the target light 1302.

A feature of the wavefront sensor 1300 is that the relay optical system 1308 includes a negative lens in one or both of a front group 1308A and a rear group 1308B, and is constituted of an afocal optical system in which the principal point spacing d58 between the front group 1308A and the rear group 1308B is larger than the optical total length a58 of the relay optical system 1308. Thereby, as compared with a case where the relay optical system 1308 does not include a negative lens, it is possible to increase the working distance while maintaining the optical total length, or to shorten the optical total length while maintaining the working distance. Therefore, even when the optical total length of the relay optical system 1308 cannot be made sufficiently large due to reasons such as space constraints, the working distance can be made large to a certain extent.

In the present embodiment, the afocal optical system constituting the relay optical system 1308 includes a front group 1308A on which the target light 1302 is incident, and a rear group 1308B on which the target light 1302 transmitted through the front group 1308A is incident. The front group 1308A includes the positive lens L5 on which the target light 1302 is incident, and the negative lens L6 on which the target light 1302 transmitted through the positive lens L5 is incident. The rear group 1308B includes the negative lens L7 on which the target light 1302 transmitted through the front group 1308A is incident, and the positive lens L8 on which the target light 1302 transmitted through the negative lens L7 is incident. Here, the "positive lens" refers to a convex lens and other lenses having positive power, and the "negative lens" refers to a concave lens and other lenses having negative power.

In the present embodiment, the relay optical system 1308 is constituted of the positive lens L5, the negative lens L6, the negative lens L7, and the positive lens L8; however, the present invention is not limited thereto. That is, any afocal optical system can be used as the relay optical system 1308 as long as it is an afocal optical system that includes one or more negative lenses in one or both of the front group 1308A and the rear group 1308B and satisfies a condition that the principal point spacing d58 between the front group 1308A and the rear group 1308B is larger than the optical total length a58.

### [Embodiment 2: Ophthalmic Wavefront Sensor]

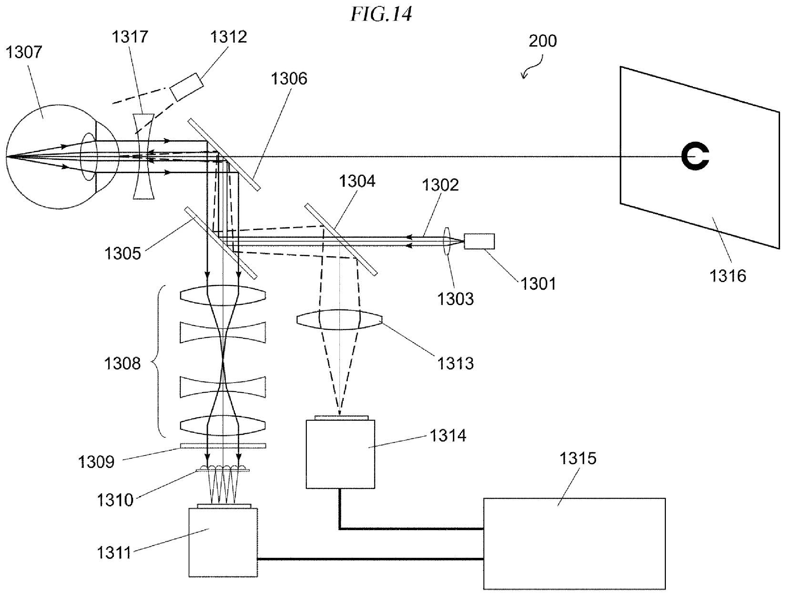

It is considered to apply the wavefront sensor according to Embodiment 1 described above to an ophthalmic wavefront sensor. Fig. 14 shows a configuration example of an open-view type ophthalmic wavefront sensor 200 in which scenery on the other side can be seen through the apparatus as described in JP 2018-501936 A and JP 2016-526985 A.

The ophthalmic wavefront sensor 200 shown in Fig. 14 is obtained by adding, to the wavefront sensor 1300 shown in Fig. 13, a measurement light source 1301, a collimating lens 1303, a dichroic mirror 1304, a beam splitter 1305, a dichroic mirror 1306, an optical filter 1309, an illumination light source 1312, a lens 1313, an image sensor 1314, and a computer 1315.

Measurement light 1302 (the target light 1302 in Embodiment 1 above) output from the measurement light source 1301 is shaped by the collimating lens 1303 into collimated light having a small beam diameter. The measurement light 1302 transmitted through the collimating lens 1303 is incident on the dichroic mirror 1304. The dichroic mirror 1304 is designed to transmit light belonging to a wavelength band including the measurement light 1302. Therefore, the measurement light 1302 transmitted through the collimating lens 1303 is transmitted through the dichroic mirror 1304.

The measurement light 1302 transmitted through the dichroic mirror 1304 is incident on the beam splitter 1305. The beam splitter 1305 is designed to reflect a part of incident light and transmit a part of the incident light. Therefore, a part of the measurement light 1302 transmitted through the dichroic mirror 1304 is reflected by the beam splitter 1305. The measurement light 1302 reflected by the beam splitter 1305 is incident on the dichroic mirror 1306. The dichroic mirror 1306 is designed to reflect light belonging to a wavelength band including the measurement light 1302. Therefore, the measurement light 1302 reflected by the beam splitter 1305 is reflected by the dichroic mirror 1306. The measurement light 1302 reflected by the dichroic mirror 1306 is focused on the fundus of the subject.

The dichroic mirror 1306 is designed to transmit visible light. For this reason, the subject can view a distant fixation target 1316 through the dichroic mirror 1306.

The measurement light 1302 incident on an eyeball 1307 from the pupil is diffusely reflected at each point on the fundus. The measurement light 1302 diffusely reflected on the fundus is incident on the dichroic mirror 1306. The dichroic mirror 1306 reflects the measurement light 1302 diffusely reflected on the fundus. The measurement light 1302 reflected by the dichroic mirror 1306 is incident on the beam splitter 1305. The beam splitter 1305 transmits a part of the measurement light 1302 reflected by the dichroic mirror 1306.

The measurement light 1302 transmitted through the beam splitter is incident on the relay optical system 1308. Functions of the relay optical system 1308, the microlens array 1310, the image sensor 1311, and the computer 1315 are as already described with reference to Fig. 13. Note that, in the present embodiment, the optical filter 1309 is added between the relay optical system 1308 and the microlens array 1310. By appropriately selecting the optical filter 1309, it is possible to set only a specific wavelength component of the measurement light 1302 as a measurement target, or to set only a polarization component of the measurement light 1302 as a measurement target.

The ophthalmic wavefront sensor 200 according to the present embodiment is not of a structure in which the apparatus is brought into close contact with an eye. Therefore, it is preferable that the ophthalmic wavefront sensor 200 includes an observation optical system for aligning the optical axis of the ophthalmic wavefront sensor 200 with the optical axis of the eyeball 1307. The observation optical system is a configuration for displaying, on an external monitor, an image in which a pupil, an eyelid, and the like in a front portion of the eyeball 1307 are included within an angle of view. By using the observation optical system, an operator can perform an operation (alignment operation) of matching a center of the pupil, which is the optical axis of the eyeball 1307, with the optical axis of the ophthalmic wavefront sensor 200. Such an observation optical system is often built in ophthalmic examination equipment.

In the ophthalmic wavefront sensor 200, the illumination light source 1312, the dichroic mirror 1306, the beam splitter 1305, the dichroic mirror 1304, the lens 1313, and the image sensor 1314 constitute the observation optical system. The observation optical system acts on illumination light reflected or scattered on a surface of the eyeball to form an image of the eyeball on the other image sensor.

The illumination light source 1312 irradiates, onto the surface of the eyeball 1307 (represented by a front surface of the cornea in the drawing), observation light having a wavelength different from that of the measurement light 1302. As the illumination light source 1312, for example, an LED can be used. In Fig. 14, the observation light is represented by a dotted line.

Illumination light output from the illumination light source 1312 is reflected on the surface of the eyeball 1307 and is incident on the dichroic mirror 1306. The dichroic mirror 1306 is designed to reflect, in addition to light in the wavelength band to which the measurement light 1302 belongs, light in a wavelength band to which the illumination light belongs. The illumination light reflected on the surface of the eyeball 1307 is reflected by the dichroic mirror 1306. The illumination light reflected by the dichroic mirror 1306 is incident on the beam splitter 1305. The beam splitter 1305 reflects a part of the illumination light reflected by the dichroic mirror 1306. The illumination light reflected by the beam splitter 1305 is incident on the dichroic mirror 1304. The dichroic mirror 1304 is designed to reflect light in the wavelength band to which the illumination light belongs. The illumination light reflected by the dichroic mirror 1304 is incident on the image sensor 1314 via the lens 1313. Thereby, an image of the surface of the eyeball 1307 is formed on the image sensor 1314.

The image sensor 1314 generates an image representing the image of the surface of the eyeball 1307. The computer 1315 displays the image generated by the image sensor 1314 on a display which is not shown. The computer 1315 may execute processing for calculating a curvature of the cornea with reference to the image generated by the image sensor 1314.

As described above, the relay optical system 1308 is constituted of the afocal optical system that includes a negative lens in one or both of the front group and the rear group, and in which the principal point spacing d58 between the front group and the rear group is larger than the optical total length a58 of the relay optical system 1308. For this reason, the working distance -s5 can be made longer than that of a conventional ophthalmic wavefront sensor. For this reason, in the ophthalmic wavefront sensor 200, the dichroic mirror 1306 for bending optical paths of the measurement light and the observation light, and the beam splitter 1305 and the dichroic mirror 1306 for separating the measurement light and the observation light are disposed in a space generated on the object side of the relay optical system 1308. Thereby, it is possible to implement the compact ophthalmic wavefront sensor 200.

With respect to the ophthalmic wavefront sensor 200, it is possible to consider several variations by changing a part of the optical system.

The beam splitter 1305 can be replaced with a polarizing beam splitter. In this case, a light source (such as a laser) whose output light is linearly polarized is used as the measurement light source 1301, or the measurement light 1302 is made into linearly polarized light using a polarizing plate. In this case, a polarizing beam splitter 1305' is designed to reflect the linearly polarized light output from the measurement light source 1301.

Light diffusely reflected on the fundus and the surface of the eyeball 1307 is diffusely reflected light, and therefore is unpolarized in any case. That is, a part of the light diffusely reflected on the fundus and the surface of the eyeball 1307 is transmitted through the polarizing beam splitter 1305', and a part thereof is reflected by the polarizing beam splitter 1305'. Thereby, the beam splitter 1305 can be caused to function similarly to a beam splitter in which reflectance and transmittance are controlled independently of polarization.

Further, the illumination light source 1312 can also be designed to project an arbitrary pattern (a specific drawing or a specific point image) onto the cornea. Thereby, an image is formed on a corneal surface, and a radius of curvature of the cornea can be analyzed from the pattern of the image. Such a mechanism for analyzing the radius of curvature of the cornea is a function widely implemented in an apparatus that measures diopter of an eye and wavefront aberration, and it is also possible to implement such a function in the ophthalmic wavefront sensor 200.

Further, since the ophthalmic wavefront sensor 200 can be easily downsized, it can also be built into another apparatus. For example, the fixation target 1316 does not need to be an actual fixation target at a far distance, and may be an optically projected image. Also in the ophthalmic wavefront sensor 200, it is possible to adopt a configuration having an internal fixation target like an autorefractometer.

Further, an apparatus that presents a fixation target so as to be optically at a far distance by using, for example, a concave mirror is referred to as space-saving chart (or space saving chart), and is widely used mainly in eyeglass shops. The fixation target 1316 may be such a space-saving chart.

Further, since the ophthalmic wavefront sensor 200 can secure a long working distance, it is possible to secure a wide space between the objective lens of the apparatus and an eye. For this reason, as shown in Fig. 14, an eyeglass lens 1317 can be placed between the eyeball 1307 and the dichroic mirror 1306. The eyeglass lens 1317 may be an eyeglass lens worn by the subject, or may be an optometric lens used for examination in an ophthalmology clinic or an eyeglass shop. By performing such an examination, it is possible to quantify whether diopter correction is correctly performed by the eyeglass lens or the optometric lens. Further, an apparatus that performs an examination by mechanically exchanging the optometric lens is also widely used, and it is also possible to integrate the ophthalmic wavefront sensor 200 with such an apparatus.

### [Summary]

### (Aspect 1)

A wavefront sensor for measuring wavefront aberration of target light includes:
an afocal optical system that relays the target light, the afocal optical system including one or more negative lenses;
a microlens array that acts on the target light transmitted through the afocal optical system to form a focal spot group; and
an image sensor that generates an image representing the focal spot group formed by the microlens array.

According to the above configuration, it is possible to increase a working distance of an afocal optical system that satisfies a rear imaging condition while maintaining an optical total length of the afocal optical system, or to shorten the optical total length of the afocal optical system while maintaining the working distance of the afocal optical system that satisfies the rear imaging condition.

### (Aspect 2)

According to Aspect 2, in the wavefront sensor according to Aspect 1, the afocal optical system includes a front group and a rear group, and
a principal point spacing d' between the front group and the rear group is longer than an optical total length a of the afocal optical system.

According to the above configuration, it is possible to increase a working distance of an afocal optical system that satisfies a rear imaging condition while maintaining the optical total length a of the afocal optical system, or to shorten the optical total length a of the afocal optical system while maintaining the working distance of the afocal optical system that satisfies the rear imaging condition.

### (Aspect 3)

According to Aspect 3, in the wavefront sensor according to Aspect 2, the front group includes: a first lens that is a positive lens on which the target light is incident, and a second lens that is the negative lens on which the target light transmitted through the first lens is incident,
the rear group includes: a third lens that is the negative lens on which the target light transmitted through the front group is incident, and a fourth lens that is a positive lens on which the target light transmitted through the third lens is incident,
a composite focal length of the front group is defined as f1' and a composite focal length of the rear group is defined as f2', d' = f1' + f2' being satisfied, and
the principal point spacing d' between the front group and the rear group is longer than a principal point spacing between the first lens and the fourth lens, that is, the optical total length a.

According to the above configuration, it is possible to implement, with a simple configuration, an afocal optical system that can increase the working distance satisfying the rear imaging condition while maintaining the optical total length a, or can shorten the optical total length a while maintaining the working distance satisfying the rear imaging condition.

### (Aspect 4)

According to Aspect 4, in the wavefront sensor according to any one of Aspects 1 to 3, the target light is light from an eyeball, and
the wavefront sensor further includes a dichroic mirror that transmits visible light and guides the visible light to the eyeball, and reflects the target light having a wavelength different from a wavelength of the visible light and guides the target light to the afocal optical system.

According to the above configuration, it is possible to implement an open-view type ophthalmic wavefront sensor.

### (Aspect 5)

According to Aspect 5, the wavefront sensor according to Aspect 4 further includes:
another image sensor;
an observation optical system for acting on illumination light reflected or scattered on a surface of the eyeball to form an image of the eyeball on the another image sensor; and
a beam splitter that guides at least a part of the target light reflected by the dichroic mirror to the afocal optical system and guides, to the observation optical system, at least a part of the illumination light that is reflected or scattered on the surface of the eyeball and is reflected by the dichroic mirror.

According to the above configuration, it is possible to add, to the open-view type ophthalmic wavefront sensor, a function of observing the surface of the eyeball.

### [Supplementary Note]

The present invention is not limited to the respective embodiments described above, and various modifications can be made within the scope recited in the claims, and embodiments obtained by appropriately combining technical means disclosed in the respective different embodiments are also included in the technical scope of the present invention.

## Claims

1. A wavefront sensor for measuring wavefront aberration of target light, the wavefront sensor comprising:
an afocal optical system (1308) that relays the target light, the afocal optical system (1308) including one or more negative lenses;
a microlens array (112, 402, 1310) that acts on the target light transmitted through the afocal optical system (1308) to form a focal spot group; and
an image sensor (113, 403, 1311) that generates an image representing the focal spot group formed by the microlens array (112, 402, 1310).

2. The wavefront sensor according to claim 1, wherein
the afocal optical system (1308) includes a front group and a rear group, and
a principal point spacing d' between the front group and the rear group is longer than an optical total length a of the afocal optical system (1308).

3. The wavefront sensor according to claim 2, wherein
the front group includes: a first lens that is a positive lens on which the target light is incident, and a second lens that is the negative lens on which the target light transmitted through the first lens is incident,
the rear group includes: a third lens that is the negative lens on which the target light transmitted through the front group is incident, and a fourth lens that is a positive lens on which the target light transmitted through the third lens is incident,
a composite focal length of the front group is defined as f1' and a composite focal length of the rear group is defined as f2', d' = f1' + f2' being satisfied, and
the principal point spacing d' between the front group and the rear group is longer than a principal point spacing a between the first lens and the fourth lens.

4. The wavefront sensor according to any one of claims 1 to 3, wherein
the target light is light from an eyeball, and
the wavefront sensor further includes a dichroic mirror that transmits visible light and guides the visible light to the eyeball, and reflects the target light having a wavelength different from a wavelength of the visible light and guides the target light to the afocal optical system (1308).

5. The wavefront sensor according to claim 4, further comprising:
another image sensor (1314);
an observation optical system for acting on illumination light reflected or scattered on a surface of the eyeball to form an image of the eyeball on the another image sensor (1314); and
a beam splitter that guides at least a part of the target light reflected by the dichroic mirror to the afocal optical system (1308) and guides, to the observation optical system, at least a part of the illumination light that is reflected or scattered on the surface of the eyeball and is reflected by the dichroic mirror.
